# EUROPEAN PATENT APPLICATION

(11) **EP 4 697 003 A1**
(43) Date of publication of application: **18.02.2026**
(21) Application number: 25187466.5
(22) Date of filing: 04.07.2025
(51) Int. Cl.: G01N 1/22, G01N 33/569

(54) **METHOD AND SYSTEM FOR DETERMINING ALLERGENIC PARTICLES IN AIR**

(30) Priority: 08.08.2024 FI 20245998
(71) Applicant: Oy Fiseca Ltd, 00100 Helsinki (FI)
(72) Inventor: Seppäläinen, Erkki, 02700 Kauniainen (FI); Partanen, Tuomo, 1268 Luxembourg (LU)
(74) Representative: Moosedog Oy

(57) **Abstract**

Disclosed is a method and system for determining allergenic particles in an air of a building. The method includes collecting a volume of air, counting particles in the air, filtering the air, measuring an amount of the collected air, calculating a particle concentration in the collected volume of air based on particle quantity and the amount of the collected air, dissolving the collected particles in a solvent to obtain dissolved particles, separating a first volume and a second volume from the dissolved particles for decomposing the dissolved particles, measuring a glucose concentration in the first volume, facilitate coupling of the decomposed particles in the second volume to fluorophore tracers and measuring fluorescent emission from the decomposed particles by exposing the decomposed particles to fluorophore tracers, and determining allergenic particles in the collected air based on the glucose concentration, the fluorescent emission, and reference data.

## Description

### TECHNICAL FIELD

The present disclosure relates to allergenic particle detection in air. The present disclosure also relates to a method and a system for determining allergenic particles in directly from air within an environment (such as a room in a building).

### BACKGROUND

Existing methods for detecting particles (such as allergen particles, fungal particles, pollen, dander, and so on) in an environment may involve extracting a sample, which is not specific to the environment (such as a building). The sample is usually extracted from a floor or a wall of the building. Once the sample is extracted, sampling and/or analytical techniques may be employed for analysis of the sample for determination of the allergen particles, fungal particles, pollen, and so on, from the extracted sample. The sampling and/or analytical techniques provide comprehensive data on the presence, concentration, and identification of various particles in the air, which may be crucial for environmental monitoring, health assessments, and regulatory compliance. The sampling techniques involve separating particles onto culture media or adhesive surfaces based on particle size, usage of centrifugal force for separating particles based on the particle size, and so on. The separated particles may be collected by impacting onto a sticky/adhesive surface or culture medium or captured in a collection vessel.

The analytical techniques include microscopic analysis, culture-based analysis, molecular analysis, immunological analysis, chemical analysis, and so on. In microscopic analysis, light microscopy is used to identify and count pollen, spores, and other allergenic or fungal particles that have been collected on slides, or electron microscopy is used for obtaining detailed images of particles for morphological identification. In culture-based analysis, articles collected on the culture media are incubated, and resulting colonies are identified and counted, or selective media is utilized to target specific types of fungi or bacteria. Molecular analysis includes DNA sequencing, Polymerase Chain Reaction (PCR), or quantitative PCR (qPCR). In DNA sequencing, particles may be identified by sequencing specific genetic markers. In PCR, specific DNA sequences are amplified to identify and quantify microorganisms. In qPCR, amount of specific DNA sequences may be measured for obtaining quantitative data on microbial load. Immunological analysis involves detection of specific proteins or antigens from allergens or microbes, or performance of rapid tests for detecting specific allergens or microbial antigens. Chemical analysis involves identifying and quantifying volatile organic compounds (VOCs) in the particles (such as mold) or detecting specific chemical markers from allergens or microbial metabolites.

However, there are issues with each of the sampling and analytical techniques. For example, in scenarios of usage of sampling technique where particle concentration is high, there are chances of overloading, which may lead to inaccuracies in particle counts or particle identification. In some other scenarios, high-impact forces may cause fragmentation of delicate particles like fungal spores, leading to underestimation, liquid media may evaporate during prolonged sampling, affecting particle capture efficiency, adhesives used to capture particles affect recovery and identification of certain types of particles, or smaller particles may not be effectively captured and, thus, may be lost. In scenarios, where analytical technique is used, there are challenges in distinguishing morphologically similar particles. Furthermore, issues may arise due to factors such as limitations in ability to culture only viable organisms which prevents detection of non-viable allergenic particles, contamination of samples with extraneous DNA which leads to obtaining false positives, interference with PCR amplification due to environmental inhibitors in air samples.

Furthermore, there are other methods for detecting allergenic particles, fungal particles, and so on, which use Petri dish. However, such methods of particle detection may be time consuming.

Therefore, considering the foregoing discussion, there exists a need to overcome the aforementioned drawbacks.

### SUMMARY

The aim of the present disclosure is to provide a method and a system for determining allergenic particles in air within an environment (such as a building). The method and system allows constant monitoring of quality of the air in a room of the building based on a determined concentration of the allergenic particles in air. The monitoring may allow determining causes of health symptoms which may be triggered due to breathing air that is contaminated by specific types of pollutants (such as allergenic particles, fungal particles, mold, inorganic dust, animal dander, pollen, or the like) and taking measures to prevent the occurrence of the health symptoms. The concentration of the allergenic particles may be determined by measuring glucose concentration in a volume of dissolved particles and fluorescent emission from decomposed particles obtained from the volume of the dissolved particles. The aim of the present disclosure is achieved by usage of the provided method and the system for determining allergenic particles in the air within the environment (i.e., the building) as defined in the appended independent claims to which reference is made to. Advantageous features are set out in the appended dependent claims.

Throughout the description and claims of this specification, the words "*comprise*", "*include*", "*have*", and "*contain*" and variations of these words, for example "*comprising*" and "*comprises*", mean "*including but not limited to*", and do not exclude other components, items, integers, or steps not explicitly disclosed also to be present. Moreover, the singular encompasses the plural unless the context otherwise requires. In particular, where the indefinite article is used, the specification is to be understood as contemplating plurality as well as singularity, unless the context requires otherwise.

### BRIEF DESCRIPTION OF THE DRAWINGS

FIG. 1 is a schematic diagram of a system for determining allergenic particles in air within an environment, according to an embodiment of the present disclosure; and
FIG. 2 depicts steps of a method for determining allergenic particles in air within an environment, in accordance with an embodiment of the present disclosure.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

In a first aspect, the present disclosure provides a method for determining allergenic particles in an air of a building, the method comprising:
collecting a volume of air from a room of a building;
filtering the collected volume of air to collect particles having size smaller than 40 µm;
counting the collected particles using an optical particle counter, wherein the collecting of the volume of air is stopped, when a particle quantity selected from at least one of is counted:
   quantity of particles from 1000 up to 500 000 having a size in a range from 20 µm up to 40 µm,
   quantity of particles from 5000 up to 2 000 000 having a size in a range from 10 µm up to 20 µm, and
   quantity of particles from 20 000 up to 10 000 000 having a size in a range from 0.1 µm up to 20 µm;
measuring an amount of the collected volume of air;
calculating a particle concentration in the collected volume of air based on the particle quantity and the amount of the collected volume of air;
dissolving the collected particles in a solvent to obtain dissolved particles;
separating a first volume from the dissolved particles for decomposing at least part of the structure of the dissolved particles;
measuring a glucose concentration in the first volume of the dissolved particles;
separating a second volume from the dissolved particles;
decomposing the second volume of the dissolved particles to obtain decomposed particles to allow coupling of the decomposed particles to one or more specific fluorophore tracers;
measuring a fluorescent emission from the decomposed particles by exposing the decomposed particles to one or more specific fluorophore tracers and to excitation light suitable for the selected fluorophore(s); and
determining allergenic particles in the collected volume of air by:
   comparing the measured glucose concentration with an existing reference glucose concentration to determine a proportion of organic particles,
   comparing the measured fluorescent emission with an existing reference fluorescent emission data to determine a proportion of fungal particles comprised in the organic particles, and
   detecting a proportion of inorganic dust based on the collected particles size distribution and particle number, the proportion of organic particles.

In a second aspect, the present disclosure provides a system of determining allergenic particles in an air of a building, the system comprising:
a collecting means for collecting a volume of air from a room of a building, the collecting means further comprising a measurement unit for measuring an amount of the collected volume of air;
a fibre filter membrane for filtering the collected volume of air to collect particles having size smaller than 40 µm;
an optical particle counter for counting the collected particles, the optical particle counter connected to the collecting means, wherein the collecting means are configured to collect the volume of air until a particle quantity selected from at least one of is counted:
   quantity of particles from 1000 up to 500 000 having a size in a range from 20 µm up to 40 µm,
   quantity of particles from 5000 up to 2 000 000 having a size in a range from 10 µm up to 20 µm, and
   quantity of particles from 20 000 up to 10 000 000 having a size in a range from 0.1 µm up to 10 µm;
a means for collecting particles from the fibre filter membrane;
a vessel for dissolving the collected particles in a solvent to obtain dissolved particles;
a separator unit for separating a first volume of the dissolved particles in a first sample cuvette and a second volume of the dissolved particles in a second sample cuvette;
a glucose concentration measuring device for measuring a glucose concentration in the first cuvette;
a fluorescent emission measuring device for measuring fluorescent emission from the second cuvette;
a processing unit connected to the measurement unit, the optical particle counter, the glucose concentration measuring device, the fluorescent emission measuring device, wherein the processing unit is configured to:
   calculate a particle concentration in the collected volume of air based on the particle quantity and the amount of the collected volume of air, and
   determine allergenic particles in the collected volume of air by:
      comparing the measured glucose concentration with an existing reference glucose concentration to determine a proportion of organic particles,
      comparing the measured fluorescent emission with an existing reference fluorescent emission data to determine a proportion of fungal particles comprised in the organic particles, and
      detecting a proportion of inorganic dust based on the collected particles size distribution and particle number, the proportion of organic particles.

The present disclosure provides the aforementioned first aspect and the aforementioned second aspect to facilitate determination of causes of health symptoms based on an analysis of an air sample in an environment such as a room in the building. The air sample is analysed for determining whether the air is contaminated and whether the contamination in the air is causing the health symptoms in persons in the room of the building. The contamination in the air is determined based on detection of fungal particles, allergenic particles, mold, inorganic dust, animal dander, pollen, and so on, in the analysed air. In general, particles with size above 40 µm (micrometres), are likely to be caught in the human respiratory system prior to the particles reaching the lungs. However, smaller particles (i.e., particles smaller than 40 µm) may easily reach the lungs and cause health issues or symptoms. Therefore, it is crucial to determine concentration of the smaller particles. The smaller particles are usually allergenic particles, fungal particles, mold, inorganic dust, animal dander, pollen, and so on. The detection of the smaller particles and a constant monitoring of their concentration allows determining whether the quality of the air in the room of the building is optimum enough to allow persons in the room to adopt of precautionary measures to minimize health hazards.

For detection of allergenic particles (including fungal particles, allergenic particles, mold, inorganic dust, animal dander, pollen, and so on) in the air, a volume of air is collected from the room of the building. The number of fungal particles or spores in the air can be determined directly from the air, based on sucking of the air using a collecting nozzle, a tube, or a hose, at a rate of 10 l/min (liter per minute). The air suction may be maintained with an air blower. The collection nozzle/tube/hose and the air blower may be parts of an air collection line whereby the air blower follows the collection nozzle, tube, or hose. The air suction rate may be monitored either with a direct flow measurement or indirectly to determine or measure an actual flow during collection of a sample of air. Optionally, the volume of air is collected at a rate from 2 l/min up to 200 l/min. The rate may vary depending on filtering and particle collection means. Additionally, an inlet (such as the collection nozzle/tube/hose) of the air collection line may vary in shape and size. Optionally, in some of the embodiments, after the inlet of the air collection line, a coarse particle filter is attached. The coarse filter ensures that the sucked air is filtered from coarse particles. In an embodiment, the coarse particle filter can be a polyester filter mat, which can remove dust, textile fibers, hair, and so on, from the air sample that is to be analyzed.

The collected volume of air is filtered to ensure that particles having size smaller than 40 µm are collected. The coarse filter may filter particles with size larger than 100 µm. This is because size of the fungal particles usually falls in a range from 1-40 µm. It is beneficial that the coarse particle filter does not collect particles of size that are smaller than 40 µm. This is because it is unlikely that fungal particles of size greater than 40 µm is present in the collected volume of air. In an embodiment, the particles in the collected air that have passed through the coarse filter are counted using the optical particle counter. The optical particle counter is operable to measure the particle count and size distribution of the particles. The measurement may yield particle concentration in different size classes in the collected air sample (i.e., the collected volume of air) when an air suction rate is known. The particles with sizes in the range 1-40 µm may be used as a criterion to determine the quantity or count of particles in the collected air sample.

In an embodiment, the collected sample of air in the air collection line is fed through a fiber filter membrane to collect most of the particles with sizes falling in the 1-40 µm. The optical particle counter The fiber filter membrane may be situated on a removable filter holder in the air collection line to ease a process of changing the filter without contamination. The fiber filter membrane may be selected such that it effectively collects particles with sizes falling in a certain range that does not cause excessive pressure-drop in the air collection line when flow rate (air sucking rate) is close to 10 l/min. The air flow through the fiber filter membrane may be maintained for pre-set time or until pre-set quantity of particles in size range from 1-40 µm has passed through the optical particle sensor (i.e., optical particle counter). After the fungal particle collection is ready, the fiber filter membrane is separated from the air collection line for initiating a following processing phase. If measurements are performed in a sequence, a new fiber filter membrane may be placed in the filter holder in the air collection line and a new sample (air) collection is started. The change of the filter may be automatized, which allows collection of a batch of air sample collections.

The amount of the collected volume of air is measured to determine a concentration of particles in the collected volume of air. This is because the particle concentration in the collected volume of air is calculated based on the particle quantity (i.e., the count of particles of sizes in different size classes) and the collected volume of air. The determination of the concentration of fungal particles or allergenic particles in the collected volume of air requires collecting a certain quantity of fungal particles or allergenic particles. For collection of the certain quantity of fungal particles, a certain volume of air is required to be collected. Typically, the collection volume of air may be required to fall in a range of 100-20,000 l to yield representative quantity of fungal particles.

By use of the optical particle counter, a cumulative quantity (i.e., count) of particles falling in certain size distributions in the collected volume of air flow can be determined. The collection of the volume of air is continued till it is ensured that a representative quantity (i.e., count) of particles in size range 1-40 µm has been collected. The optical particle counter allows counting number of particles and classifying particles into different groups representing particle sizes. This allows counting particles of specific sizes. When it is determined, using the optical particle counter, that the representative quantity (i.e., count) of particles have been collected, the collection of the volume of air can be stopped. The volume of air may be collected for a period that falls in the range of 10-600 minutes. The collection of the volume of air is stopped when count of particles, of size in the range 0.1-10 µm, is in a range of 20,000-10,000,000. The collection of the volume of air is also stopped when count of particles, of size in the range 10-20 µm, is in a range of 5,000-2,000,000. The collection of the volume of air is also stopped when count of particles, of size in the range 20-40µ m, is in a range of 1,000-500,000. The mass of the collected particles may fall in the range of 5-200 µg (micrograms). It may be noted that for smaller particles, i.e., particles with sizes in the range 0.1-10 µm, a greater number of particles is required to be collected (compared to particles with sizes greater than 20 µm) for an efficient analysis of the collected volume of air. An efficient analysis results in an accurate determination of concentration of allergenic particles, fungal particles, pollen, mold, and so on, in the air. The representative quantity (i.e., count) of particles required to be collected and the mass of the collected particles may vary depending on quality of air outside the building, quality of air inside the building, and air ventilation means in the building.

The collected particles are dissolved in the solvent to obtain the dissolved particles. In an embodiment, the collected particles may be inserted in a cuvette with a suitable solvent to dissolve the collected particles from a filter (such as the coarse filter or the fiber filter membrane) to the solvent. Optionally, a volume of the solvent is in a range of 0.1-5 ml (milliliters). The required volume of the solvent depends on a type of the filter and its size. Optionally, the collected particles may be dissolved in the solvent that is selected from at least one of: a water-based solution, or a dimethyl sulfoxide-based solution. The water-based solution is used for dissolving particles that are water soluble whereas the dimethyl sulfoxide-based solution is used for dissolving particles that are insoluble in water. Thereafter, a sample volume of a liquid solution, i.e., the solvent with the dissolved particles, may be injected in two separate processing cuvettes. The first volume is separated from the dissolved particles (i.e., the liquid solution comprising the solvent and the dissolved particles) and injected in a first cuvette. Similarly, the second volume is separated from the dissolved particles (i.e., the liquid solution comprising the solvent and the dissolved particles) and injected in a second cuvette. Optionally, a volume range for at least one of the first volume of the dissolved particles and the second volume of dissolved particles is 0.01-2 ml.

The first volume is separated for decomposing at least part of a structure of the dissolved particles in the first volume and measuring a glucose concentration in the first volume of the dissolved particles. The injection of the first volume in the first cuvette results in decomposition of allergenic and fungal particles (the dissolved particles) partly into glucose. The decomposition may be triggered based on an exposure of the first volume to a certain temperature, a reduced potential of hydrogen (pH), and lytic enzymes. Optionally, decomposing of the first volume of the dissolved particles may be carried out at a temperature that falls in a range of 40-85 °C (degree centigrade) and at a pH that falls in a range of 0-3. The fungal particles may decompose within a period that falls in a range of 20-1200 seconds. Optionally, during the decomposing of the first volume of fungal particles (i.e., the dissolved particles) lytic enzymes are added. The lytic enzymes may be selected from at least one of: a chitinase, a glucanase, a proteinase, or a peroxidase. The glucose (into which allergenic and fungal particles (i.e., the dissolved particles) have decomposed) is measured using a glucose concentration measurement device. In an embodiment, a portion of the first volume may be transferred to the glucose measurement device using a pipette or by suction means. The portion transferred is within a range of 1-500 µl (microliters). An exact portion of transfer may depend on the glucose measurement device. The glucose measurement device may measure the glucose concentration. The measurement of the glucose concentration allows detecting organic (allergenic and fungal) particles amongst other types of particles (such as inorganic dust).

The second volume is separated for decomposing the dissolved particles in the second volume and enable coupling of the decomposed particles to one or more specific fluorophore tracers. The injection of the second volume in the second cuvette results in a partial decomposition of fungal particles (the dissolved particles). The partial decomposition may expose molecular structures of the fungal particles dissolved in the second volume. The molecular structures of the dissolved fungal particles couple with the one or more specific fluorophore tracers that may be added to the second volume. Optionally, decomposing of the second volume of the dissolved particles (i.e., decomposing of fungal particles in the separated second volume) is carried out at a temperature that falls in a range of 20-60 °C and at a pH that falls in a range of 9-11. The decomposition of the dissolved particles (i.e., the fungal particles) takes place within a period that falls in a range of 20-1200 seconds. It is to be noted that the molecular structures of the dissolved fungal particles are uncharacteristic in inorganic materials. Thus, fungal particles may be detected amongst organic particles based on the decomposition of the dissolved particles in the second volume.

Optionally, the second volume of the dissolved particles, i.e., the fungal particles that are dissolved in the second volume, is decomposed using an aqueous formaldehyde solution with concentration in a range of 3-6%. In an embodiment, concentration of fluorophores added to the second volume lies in a range of 0.1-5%. Optionally, the fluorophores are added to the second volume during decomposing of the particles (i.e., the fungal particles) dissolved in the second volume. The fluorophores are selected from at least one of: calcofluor, solophenyl, pontamine. The addition of the fluorophores results in emission of light due to fluorescence when exposed to excitation light specific to selected fluorophore.

In an embodiment, a certain portion of the second volume, after the decomposition of fungal particles dissolved in the second volume, is transferred to a fluorescent measurement device using a pipette or by suction means. The portion of transferred second volume may lie in a range of 1-1000 µl. An exact proportion of the transferred second volume depends on the fluorescence measurement device. The emission of light due to the fluorescence may be measured using a luminance detector by exposing the second volume to ultraviolet A (UVA) radiation. The second volume that is exposed to UVA radiation has already been exposed to a temperature falling in the range 20-60 °C, a pH falling in a range of 9-11, and the aqueous formaldehyde solution with concentration in the range of 3-6%. The UVA radiation is generated by an emitter.

A fluorescent emission from decomposed particles in the second volume is measured by exposing the decomposed particles in the second volume to the one or more specific fluorophore tracers and to excitation light suitable for the selected fluorophore. In an embodiment, when the second volume including calcofluor (and exposed to a temperature falling in the range 20-60 °C, a pH falling in a range of 9-11, and an aqueous formaldehyde solution with concentration in a range of 3-6%) is exposed to UVA, an excitation wavelength of the calcofluor falls in a range of 300-400 nm (nanometres) with an optimum wavelength of 365 nm. The fluorescence emission falls in a range of 400-500 nm with an optimum wavelength of 440 nm. In another embodiment, when the second volume including solophenyl (and exposed to a temperature falling in the range 20-60 °C, a pH falling in a range of 9-11, and an aqueous formaldehyde solution with concentration in a range of 3-6 %) is exposed to blue light, an excitation wavelength of the solophenyl may fall in a range of 440-520 nm with an optimum wavelength of 480 nm. The fluorescence emission falls in a range of 480-570 nm with an optimum wavelength of 530 nm.

In yet another embodiment, when the second volume including pontamine (and exposed to a temperature falling in the range 20-60 °C, a pH falling in a range of 9-11, and an aqueous formaldehyde solution with concentration in the range of 3-6%) is exposed to blue light, an excitation wavelength of the pontamine may fall in a range of 400-580 nn with the optimum wavelength of 480 nm. The fluorescence emission falls in a range of 550-610 nn with an optimum wavelength of 590 nm.

The fluorescence emission is measured using the fluorescent measurement device. In an embodiment, light amplifiers such as lamps, mirrors, lenses, light tubes, photon multipliers, and so on, are used to enhance luminance prior to fluorescence/luminance (light emission) detection. Based on the fluorescence emission wavelength based on exposure of UVA or blue light at specific excitation wavelengths of fluorophore tracers, a proportion of the fungal particles may be determined from amongst the organic particles.

Based on the glucose concentration and the fluorescence emission presence or concentration of particles such as allergenic particles, fungal particles, mold, inorganic dust, animal dander, pollen, and so on, in the air is determined. The fungal particles, mold, animal dander and pollen are organic particles which may be determined based on the glucose concentration and the proportion of the fungal particles amongst the organic particles is determined based on the fluorescence emission. For determination of the presence or concentration of the particles, a reference data is required to be obtained. The reference data may include measurements that are obtained from a volume of air and indications of presence of microbiological organisms in the volume of air. The reference data may be obtained in a manner that is scientifically sufficient and suitable. For example, the reference data may be obtained using laboratory petri dish method or Polymerase Chain Reaction (PCR) method. In an embodiment, the reference data comprises predefined measurements of glucose concentration and predefined measurement of light emission (fluorescence) for samples that have been measured using petri dish method or PCR method. The reference data includes a reference glucose concentration and a reference fluorescent emission data.

Once the reference data is obtained, allergenic particles in the collected volume of air is determined by one or more of comparing the measured glucose concentration with an existing reference glucose concentration, comparing the measured fluorescent emission with an existing reference fluorescent emission data, and detecting a proportion of inorganic dust. The comparison between the measured glucose concentration with the reference glucose concentration enables determining a proportion of organic particles. The comparison between the measured fluorescent emission (i.e., the measured wavelength of fluorescence emission for each of calcofluor, solophenyl, and pontamine) and the existing reference fluorescent emission data enables determining a proportion of fungal particles comprised in the organic particles. The proportion of inorganic dust is detected based on a collected particles size distribution (such as 0.1-10 µm, 10-20 µm, and 20-40 µm), particle number (such as 1000-500,000, 5,000-2,000,000, and 20,000-10,000,000) and the determined proportion of organic particles.

It may be noted that the detection of fungal particles, allergenic particles, mold, inorganic dust, animal dander, pollen, and so on, in the analysed volume of air allows determining quality of air in the room of the building. Since inhaling different types of organic and inorganic particles, especially of sizes less than 40 µm, can lead to different types of health symptoms in persons who may be present in the room of the building, knowledge of concentration of the allergenic particles and organic particles (including fungal particles) allows predicting specific types of health hazards that the persons in the room are vulnerable to. This allows the persons to adopt to specific precautionary measures, which allows avoiding potential complications arising from contracting the health hazards.

Optionally, a temperature of the collected volume of air is measured. The temperature can be used to characterize the air in the room of the building. This is because a concise determination of particle concentration and particle count in the analysed volume of air is actualized based on measured air temperature, relative humidity, and absolute humidity.

The present disclosure also relates to the second aspect as described above. Various embodiments and variants disclosed above, with respect to the aforementioned first aspect, apply *mutatis mutandis* to the second aspect.

The system comprises means for collecting and transmitting obtained data, such as the measured glucose concentration and the fluorescence emission, to the processor. The processor may fetch information from a database of reference data stored in a memory. The processor is operable to compare the reference data to the obtained data for determining a quantity, count, and concentration of allergenic particles, fungal particles, mold, inorganic dust, animal dander, pollen, and so on, in the collected volume of air. In an embodiment, the glucose and light emission (i.e., the fluorescence) measurement values are compared to the particle count (such as 1000-500,000, 5,000-2,000,000, and 20,000-10,000,000) and size distribution (such as 0.1-10 µm, 10-20 µm, and 20-40 µm) obtained by use of the optical particle counter.

The glucose and the light emission measurement values may be sensitive to the allergenic particles, fungal particles, mold, inorganic dust, animal dander, pollen, and so on, which information can be used to calculate a fraction of each of the allergenic particles, fungal particles, mold, inorganic dust, animal dander, pollen, and so on, from a total count of collected particles. Since the collected volume of air is filtered to ensure that particles having size smaller than 40µm are collected, it may be of importance to determine the volume of air that has flown through the coarse filter or the fibre filter membrane. For more concise results of particle concentration and particle count, air temperature, relative humidity and absolute humidity values may be used to characterise the collected volume of air. Based on the air temperature, relative humidity and absolute humidity values, the glucose concentration and light emission (fluorescence) values may be correlated with reference data, and count or concentration of each of the allergenic particles, fungal particles, mold, inorganic dust, animal dander, pollen, and so on, in the air can be determined.

In some embodiments, the system comprises a means for measuring flow of air at an air outlet of the means for collecting fungal particles. The air from which the fungal particles are separated is conducted through the air collection line to an air flow meter. An additional air filter may precede the air flow meter. The flow of air may be measured by use of a hot wire anemometer or any other suitable means (such as an air balancing hood measurement, an airflow traverse in an exhaust duct, a plot fan airflow, and so on). A volume of the air is used as a co-variable for determining quantity or count of allergenic and fungal particles in the air. The quantity may be used for determining the duration of the particle sampling.

In some embodiments, the system comprises a calibrated fan, a blower, or a centrifugal pump to suck out the air from the system. The system also comprises at least one of a thermometer for obtaining temperature data, a means for measuring relative air-water content for obtaining relative air-water content data and a means for measuring absolute air-water content for obtaining an absolute air-water content. These measurements would then also be communicated to the processor and considered in the calculation of concentration of allergenic particles in air.

In some embodiments, the system comprises an interface where results of data processing (i.e., a proportion of organic particles, a proportion of fungal particles comprised in the organic particles, and a proportion of inorganic dust) can be displayed. The interface may be a display screen, or a display that is integrated to the system or a laptop, a cellular phone, a tablet, or other devices. The results may be rendered as a graphical output that indicates a variation and a trend in change of concentration of fungal particles in a unit of air (for example, fungal particles/m³) if repetitive measurements are performed. The results are also displayed in a user interface in a simplified form that indicates whether the air is clean, endangered, polluted, or an absolute value that is representative of quality of analysed air.

In an embodiment, numerical values representative of the quality of analysed air may be transformed into a graphical representation that indicates the quality of the air. Based on variations in measurement data obtained through determination of air quality at different measurement locations, a source of a microbial damage may be located without invasion of structures of a building (environment). The system may be operable to communicate the measurement data to a centralized monitoring arrangement. The system may be further operable to send warnings to a user device if the measurement data indicates that the air quality is poor.

Optionally, the system further comprises a coarse filter before the fibre filter. The coarse filter is configured to remove (filter) particles having sizes that are larger than 100µm.

Optionally, the collecting means (used for collecting the volume of air from the room of a building) is selected from at least one of: a collecting nozzle, a tube, a hose.

Optionally, the system further comprises at least one of selected from at least one of: a thermometer, a means for measuring relative air water content, and a means for measuring absolute air-water content.

### DETAILED DESCRIPTION OF THE DRAWINGS

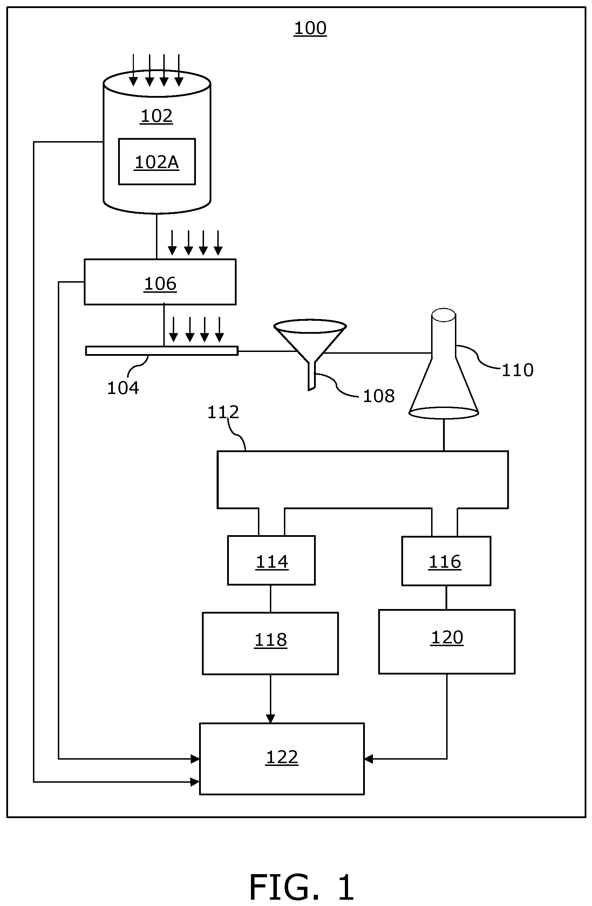

Referring to FIG. 1, there is shown a schematic diagram of a system **100** for determining allergenic particles in air within an environment (such as a room of a building), in accordance with an embodiment of the present disclosure. The system **100** comprises a collecting means **102,** a fibre filter membrane **104,** an optical particle counter **106,** a particle collection means **108,** a vessel **110,** a separator unit **112,** a first sample cuvette **114,** a second sample cuvette **116,** a glucose concentration measuring device **118,** a fluorescent emission measuring device **120,** and a processing unit **122.** The collecting means **102** collects a volume of air from the room of the building and comprises a measurement unit **102A** for measuring an amount of the collected volume of air. The collecting means **102** may be selected from amongst a collecting nozzle, a tube, or a hose. e Optionally, the collected volume of air may be initially filtered by a coarse filter (not shown) to remove particles in the collected volume of air that are having sizes larger than 100 µm. The particles that have passed through the coarse filter are counted by the optical particle counter **106.** The fibre filter membrane **104** filters the collected volume of air to ensure that particles having size smaller than 40 µm (micrometres) are collected. The optical particle counter **106** is operable to count the particles that have passed the coarse filter and are to be collected in the fibre filter membrane. The optical particle counter **106** is connected to the collecting means **102.** The collecting means **102** is operable to collect the volume of air until quantity/count of particles, having a size in a range of 20-40 µm, in the collected volume of air is in a range of 1,000-500,000. The collecting means **102** is also operable to collect the volume of air until the quantity/count of particles, having a size in a range of 10-20 µm, in the collected volume of air is in a range of 5,000-2,000,000. The collecting means **102** is also operable to collect the volume of air until the quantity/count of particles, having a size in a range of 0.1-10 µm, in the collected volume of air is in a range of 20,000-10,000,000.

The particle collection means **108** is used for collecting particles from the fibre filter membrane **104A.** The vessel **110** is used for dissolving the collected particles in a solvent to obtain dissolved particles. The separator unit **112** is used for separating a first volume of the dissolved particles in the first sample cuvette **114** and a second volume of the dissolved particles in a second sample cuvette **116.** The glucose concentration measuring device **118** is used for measuring a glucose concentration in the first sample cuvette **114.** The fluorescent emission measuring device **120** is used for measuring fluorescent emission from the second sample cuvette 116. The processing unit **122** is connected to one or more of: the measurement unit **102A,** the optical particle counter **106,** the glucose concentration measuring device **118,** and the fluorescent emission measuring device **120.**

The processing unit **122** is operable to calculate a particle concentration in the collected volume of air based on particle quantity and the amount of the collected volume of air and determine allergenic particles in the collected volume of air. The allergenic particles is determined by one or more of: comparing the measured glucose concentration with an existing reference glucose concentration to determine a proportion of organic particles, comparing the measured fluorescent emission with an existing reference fluorescent emission data to determine a proportion of fungal particles comprised in the organic particles, and detecting a proportion of inorganic dust based on the collected particles size distribution ( in range such as 0.1-10 µm, 10-20 µm, and 20-40 µm), particle number (in range such as 1000-500,000, 5,000-2,000,000, and 20,000-10,000,000), and the determined proportion of organic particles.

It may be understood by a person skilled in the art that FIG. 1 depicts a simplified schematic diagram of the system **100,** for sake of clarity, which should not unduly limit the scope of the claims herein. It is to be understood that the specific implementation of the system **100** is provided as an example and is not to be construed as limiting the scope of the claims. The person skilled in the art will recognize many variations, alternatives, and modifications of embodiments of the present disclosure.

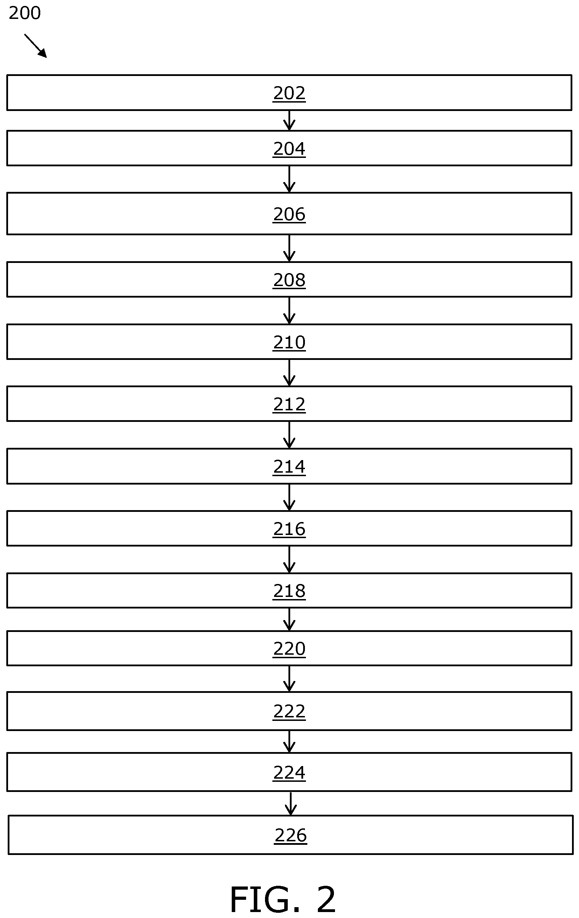

Referring to FIG. 2, depicted are steps of a method **200** for determining allergenic particles in air within an environment such as a room of a building), in accordance with an embodiment of the present disclosure. At step **202,** a volume of air is collected from the room of the building. The volume of air is collected at a rate from 2 l/min (liter per minute) up to 200 l/min. At step **204,** the collected volume of air is filtered by coarse filter **104B** to separate particles larger than 100 µm from the air stream. At step **206** the particles that have passed coarse filter **104B** are counted using the optical particle counter **106.** At step **208,** the collected volume of air is filtered by fibre filter membrane **104A** to collect particles having size smaller than 40 µm. The collecting of the volume of air is stopped if at least one of the following three conditions are satisfied. The conditions are the collected volume of air includes 1,000-500,000 particles which are of size 20-40 µm, the collected volume of air includes 5,000-2,000,000 particles which are of size 10-20 µm, and the collected volume of air includes 20,000-10,000,000 particles which are of size 0.1-10 µm. At step **210,** an amount of the collected volume of air is measured. At step**212,** a particle concentration in the collected volume of air is calculated based on the particle quantity/count (for example, 1,000-10,000,000) and the amount of the collected volume of air. At step**214**, the collected particles are dissolved in a solvent to obtain dissolved particles. The solvent may be selected from at least one of: a water-based solution and a dimethyl sulfoxide-based solution. A volume of the solvent falls in the range of 0.1-5 ml (millilitres). At step**216**, a first volume is separated from the dissolved particles for decomposing at least a part of the structure of the dissolved particles. The decomposing of the first volume of the dissolved particles is carried out at a temperature that falls in a range of 40-85 °C (degree centigrade) and at a pH that may fall in a range of 0-3. The decomposition takes place within a period that can last from 20 seconds to 1200 seconds. During the decomposing of the first volume of the dissolved particles, lytic enzymes are added. The lytic enzymes are selected from at least one of: a chitinase, a glucanase, a proteinase or a peroxidase. At step**218**, a glucose concentration in the first volume of the dissolved particles is measured. At step**220**, a second volume is separated from the dissolved particles. A volume range for at least one of the first volume of the dissolved particles and the second volume of dissolved particles is 0.01-2 ml. At step **222,** the second volume of the dissolved particles is decomposed to obtain decomposed particles. This allow coupling of molecular structures of the decomposed particles to one or more specific fluorophore tracers. The decomposing of the second volume of the dissolved particles is carried out at a temperature that falls in a range of 20-60 °C and at a pH that falls in a range of 9-11. The decomposition takes place within a period that can last from 20 seconds to 1200 seconds. The second volume of the dissolved particles is decomposed using an aqueous formaldehyde solution having a concentration from 3% up to 6%. At step **224,** a fluorescent emission from the decomposed particles is measured by exposing the decomposed particles to one or more specific fluorophore tracers and to excitation light suitable for selected fluorophores. The fluorophores are added into the second volume of the dissolved particles during the decomposition. The fluorophores are selected from at least one of: calcofluor, solophenyl, and pontamine. At step **226,** allergenic particles in the collected volume of air are determined by comparing the measured glucose concentration with an existing reference glucose concentration to determine a proportion of organic particles, comparing the measured fluorescent emission with an existing reference fluorescent emission data to determine a proportion of fungal particles comprised in the organic particles, and detecting a proportion of inorganic dust based on the a collected particles size distribution, and particle number, and the proportion of organic particles.

The aforementioned steps are only illustrative and other alternatives can also be provided where one or more steps are added, one or more steps are removed, or one or more steps are provided in a different sequence without departing from the scope of the claims herein.

## Claims

1. A method (200) of determining allergenic particles in an air of a building, the method comprising:
- collecting a volume of air from a room of a building;
- filtering the collected volume of air to collect particles having size smaller than 40 µm;
- counting the collected particles using an optical particle counter (106), wherein the collecting of the volume of air is stopped, when a particle quantity selected from at least one of is counted:
- quantity of particles from 1000 up to 500 000 having a size in a range from 20 µm up to 40 µm,
- quantity of particles from 5000 up to 2 000 000 having a size in a range from 10 µm up to 20 µm, and
- quantity of particles from 20 000 up to 10 000 000 having a size in a range from 0.1 µm up to 10 µm;
- measuring an amount of the collected volume of air;
- calculating a particle concentration in the collected volume of air based on the particle quantity and the amount of the collected volume of air;
- dissolving the collected particles in a solvent to obtain dissolved particles;
- separating a first volume from the dissolved particles for decomposing at least part of the structure of the dissolved particles;
- measuring a glucose concentration in the first volume of the dissolved particles;
- separating a second volume from the dissolved particles;
- decomposing the second volume of the dissolved particles to obtain decomposed particles to allow coupling of the decomposed particles to one or more specific fluorophore tracers;
- measuring a fluorescent emission from the decomposed particles by exposing the decomposed particles to one or more specific fluorophore tracers and to excitation light suitable for selected fluorophore(s);
- determining allergenic particles in the collected volume of air by:
- comparing the measured glucose concentration with an existing reference glucose concentration to determine a proportion of organic particles,
- comparing the measured fluorescent emission with an existing reference fluorescent emission data to determine a proportion of fungal particles comprised in the organic particles, and
- detecting a proportion of inorganic dust based on a collected particles size distribution, particle number, and the proportion of organic particles.

2. A method (200) according to claim 1, wherein the volume of air is collected at a rate from 2 l/min up to 200 l/min.

3. A method (200) according to claims 1 or 2, wherein the collected particles are dissolved in the solvent selected from at least one of: a water-based solution and a dimethyl sulfoxide-based solution.

4. A method (200) according to any of the preceding claims, wherein a volume of the solvent is from 0.1 ml up to 5 ml.

5. A method (200) according to any of the preceding claims, wherein a volume range for at least one of the first volume of the dissolved particles and the second volume of dissolved particles is from 0.01 ml up to 2 ml.

6. A method (200) according to any of the preceding claims, wherein the decomposing of the first volume of the dissolved particles is carried out
- at temperature range from 40 °C up to 85 °C;
- at pH range from 0 pH up to 3 pH; and
- during a time period from 20 seconds up to 1200 seconds.

7. A method (200) according to any of the preceding claims, wherein during the decomposing of the first volume of the dissolved particles lytic enzymes are added and wherein the lytic enzymes are selected from at least one of: a chitinase, a glucanase, a proteinase or a peroxidase.

8. A method (200) according to any of the preceding claims, wherein the decomposing of the second volume of the dissolved particles is carried out
- at temperature range from 20 °C up to 60 °C;
- at pH range from 9 pH up to 11 pH; and
- during time period from 20 seconds up to 1200 seconds.

9. A method (200) according to any of the preceding claims, wherein the second volume of the dissolved particles is decomposed using an aqueous formaldehyde solution having a concentration from 3% up to 6%.

10. A method (200) according to any of the preceding claims, wherein fluorophores are added into the second volume of the dissolved particles during decomposing and wherein the fluorophores are selected from at least one of: calcofluor, solophenyl, and pontamine.

11. A method (200) according to any of the preceding claims further comprising measuring a temperature of the collected volume of air.

12. A system (100) of determining allergenic particles in an air of a building, the system comprising:
- a collecting means (102) for collecting a volume of air from a room of a building, the collecting means further comprising a measurement unit (102A) for measuring an amount of the collected volume of air;
- a fibre filter membrane (104) for filtering the collected volume of air to collect particles having size smaller than 40 µm;
- an optical particle counter (106) for counting the collected particles, the optical particle counter connected to the collecting means, wherein the collecting means are configured to collect the volume of air until a particle quantity selected from at least one of is counted:
- quantity of particles from 1000 up to 500 000 having a size in a range from 20 µm up to 40 µm,
- quantity of particles from 5000 up to 2 000 000 having a size in a range from 10 µm up to 20 µm, and
- quantity of particles from 20 000 up to 10 000 000 having a size in a range from 0.1 µm up to 10 µm;
- a means (108) for collecting particles from the fibre filter membrane;
- a vessel (110) for dissolving the collected particles in a solvent to obtain dissolved particles;
- a separator unit (112) for separating a first volume of the dissolved particles in a first sample cuvette (114) and a second volume of the dissolved particles in a second sample cuvette (116);
- a glucose concentration measuring device (118) for measuring a glucose concentration in the first cuvette;
- a fluorescent emission measuring device (120) for measuring fluorescent emission from the second cuvette; and
- a processing unit (122) connected to the measurement unit, the optical particle counter, the glucose concentration measuring device, the fluorescent emission measuring device, wherein the processing unit is configured to:
- calculate a particle concentration in the collected volume of air based on the particle quantity and the amount of the collected volume of air; and
- determine allergenic particles in the collected volume of air by:
- comparing the measured glucose concentration with an existing reference glucose concentration to determine a proportion of organic particles,
- comparing the measured fluorescent emission with an existing reference fluorescent emission data to determine a proportion of fungal particles comprised in the organic particles, and
- detecting a proportion of inorganic dust based on the collected particles size distribution and particle number, the proportion of organic particles.

13. A system (100) according to claim 12, further comprising a coarse filter before the fibre filter, the coarse filter configured to remove particles larger than 100 µm.

14. A system (100) according to claims 12 or 13, wherein the collecting means (102) is selected from at least one of: a collecting nozzle, a tube, or a hose.

15. A system (100) according to any of the claims 12-14, further comprising at least one of: a thermometer, a means for measuring relative air water content, and a means for measuring absolute air water content.
